**Europäisches Patentamt**

**European Patent Office**

**Office européen des brevets**

(19)

(11) Veröffentlichungsnummer: **0 288 966**

**A1**

(12)

# EUROPÄISCHE PATENTANMELDUNG

(21) Anmeldenummer: **88106649.2**

(22) Anmeldetag: **26.04.88**

(51) Int. Cl.⁴: **A61B 5/10 , F16H 25/12**

(30) Priorität: **27.04.87 DE 3714015**

(43) Veröffentlichungstag der Anmeldung:
**02.11.88 Patentblatt 88/44**

(84) Benannte Vertragsstaaten:
**AT BE CH DE ES FR GB GR IT LI LU NL SE**

(71) Anmelder: **Landwehr, Ulrich M.**
**Bahnhofstrasse 8**
**D-3000 Hannover 1(DE)**

(72) Erfinder: **Landwehr, Ulrich M.**
**Bahnhofstrasse 8**
**D-3000 Hannover 1(DE)**
Erfinder: **Rackwitz, Günter**
**Berliner Allee 9**
**D-3162 Ütze/Dolbergen(DE)**

(74) Vertreter: **Döring, Roger, Dipl.-Ing.**
**Kabelkamp 20**
**D-3000 Hannover 1(DE)**

(54) **Vorrichtung zur Erzielung einer reproduzierbaren Körperhaltung.**

(57) Bei einer Vorrichtung zur Erzielung einer reproduzierbaren Körperhaltung des Menschen in freiem Stand, werden die durch das Körper-Gewicht erzeugten Kräfte auf Meßelemente übertragen. Die Vorrichtung besteht aus einer Basisplatte (1) und einer über einem Biegestab (2) mit der Basisplatte (1) verbundenen Standplatte (7), wobei an dem Biegestab (2) zumindest zwei Dehnungsmeßstreifen (9) befestigt sind. Auf der Standplatte (7) sind zwei Fußplatten (16) nebeneinander höhenverstellbar angeordnet.

Fig 1

## Vorrichtung zur Erzielung einer reproduzierbaren Körperhaltung

Die Erfindung betrifft eine Vorrichtung nach dem Oberbe-griff des Anspruchs 1.

Aus dem DE-GM 82 26 585 ist eine Vorrichtung bekannt, mit der es möglich ist, im orthopädischen Bereich bei Schäden am Bewegungsapparat innerhalb einer Therapie oder nach Operationen exakt Veränderungen nachzuweisen und zu dokumentieren, sowohl durch Fotografie als auch durch Röntgenaufnahmen. Um vergleichend messen und beurteilen zu können, ist es wichtig, bei späteren Aufnahmen stets die gleiche Körperhaltung wie bei der ersten Aufnahme zu erzielen. Bei der bekannten Vorrichtung ist die Standplatte auf mindestens zwei Meßpunkten gelagert, die entweder als Dehnungsmeßstreifen oder als Drucksensoren ausgebildet sind.

Der Nachteil dieser bekannten Vorrichtung ist darin zu sehen, daß die gleichgewichtige Haltung des Menschen nur in der Frontalebene links/rechts zu erzielen ist und nicht gleichzeitig in der Sagitalebene vorn/hinten. Die reproduzierbare Zentrierung des menschlichen Körpers auf einen Punkt ist mit dieser Vorrichtung nicht erreichbar.

Ein weiterer Nachteil dieser Vorrichtung besteht darin, daß durch die direkte Lagerung der Platte auf den Meßelementen die Anfälligkeit gegen Beschädigung relativ hoch ist, und daß für die Messung der Ungleichgewichtigkeit nur in der Frontalebene stets zwei Meßpunkte erforderlich sind. Ferner müssen sehr teure, temperaturkompensierte nach dem Trägerfrequenzverfahren arbeitende Verstärker verwendet werden.

Eine verbesserte Ausführung einer solchen Vorrichtung ist durch die DE-OS 33 01 864 bekannt geworden. Bei einem besonders vorteilhaften Ausführungsbeispiel dieser Vorrichtung sind zwei parallele Platten - eine Basisplatte und im Abstand dazu und oberhalb derselben eine Standplatte-vorgesehen, welche durch einen in der Mitte der Platte angeordneten Biegestab miteinander verbunden sind. An dem Biegestab sind Dehungsmeßstreifen befestigt, welche bei einer Biegung des Biegestabes infolge einer ungleichgewichtigen Belastung der Standplatte eine elektrische Verstärkerschaltung ansprechen, welche die ungleichgewichtige Belastung der Standplatte in Größe und Richtung auf einer Anzeige sichtbar macht. Eine solche Vorrichtung hat sich insbesondere im medizinischen Bereich z.B. für Reihenuntersuchungen auf Haltungsfehler als vorteilhaft erwiesen.

Es ist bekannt, daß man Haltungsfehler, welche aus ungleichen Beinlängen, Beckenschiefstand u.ä. herrühren, dadurch beheben kann, daß man dem Patienten z.B. orthopädisches Schuhwerk mit unterschiedlich starken Sohlen oder Einlagen verschreibt. Mit den beschriebenen bekannten Vorrichtungen ist die Feststellung des Maßes der Sohlenstärke bzw. der Höhe der Einlage sehr mühsam, ungenau und zeitaufwendig. Sie kann dadurch erreicht werden, daß man auf der Standplatte für die "anzuhebende" Seite zusätzlich Platten unterschiedlicher Dicke anordnet, auf welche der Fuß des Patienten ruht. Nach Bereitstellung eines solchen "Beinlängenausgleichs" muß der Patient neu untersucht werden.

Für derartige medizinische Untersuchungen und Dokumentationen verwendet man anstelle der Röntgenaufnahme vorteilhafterweise ein unter dem Markenzeichen "optimetric" bekanntgewordenes Verfahren, welches in der DE-PS 29 48 010 beschrieben ist. Dieses Verfahren zeichnet sich dadurch aus, daß ein Bündel aus horizontal verlaufenden Linien schräg von oben auf den Patienten projiziert wird. Hierzu können in einfacher Weise Erhebungen und Senkungen sichtbar gemacht und vermessen werden. Zum Vergleich der therapeutischen Maßnahme muß der Arzt sowohl vor als auch nach der Therapie jeweils eine Aufnahme machen und beide Aufnahmen miteinander vergleichen.

Der vorliegenden Erfindung liegt die Aufgabe zugrunde, die bekannte Vorrichtung so weiterzubilden, daß die therapeutischen Maßnahmen vorgenommen werden können, ohne daß der Patient seine Stellung wesentlich verändern muß, d.h. er die Vorrichtung nicht verlassen bzw. auch nicht einen Fuß zwecks Unterschieben einer Platte anheben muß. Weiterhin soll die Vorrichtung robust gebaut sein, d.h. auch eine stoßweise einseitige Belastung von ca. 100 kg aushalten können. Auch soll die Bauhöhe der bekannten Vorrichtung nicht überschritten werden.

Diese Aufgabe wird durch das im Kennzeichen des Anspruchs 1 Erfaßte gelöst.

Neben den sich aus der Aufgabenstellung direkt ergebenden Vorteilen zeichnet sich die erfindungsgemäße Vorrichtung noch dadurch aus, daß die Wirkung der therapeutischen Maßnahme des "Beinlängenausgleiches" direkt beobachtet und aufgezeichnet werden kann. Hier bietet sich als Aufzeichnungsmöglichekit ein Videogerät bestehend aus Videokamera, Videorekorder und Monitor an. Im Unterschied zu dem eingangs erwähnten Aufzeichnungsverfahren ist bei der Videoaufzeichnung eine Dauerprojektion der Linien erforderlich. Als vorteilhaft erweist sich darüberhinaus, daß Standbilder und deren Abspeicherung möglich ist.

Weitere vorteilhafte Ausgestaltungen der Erfindung sind in den Unteransprüchen erfaßt.

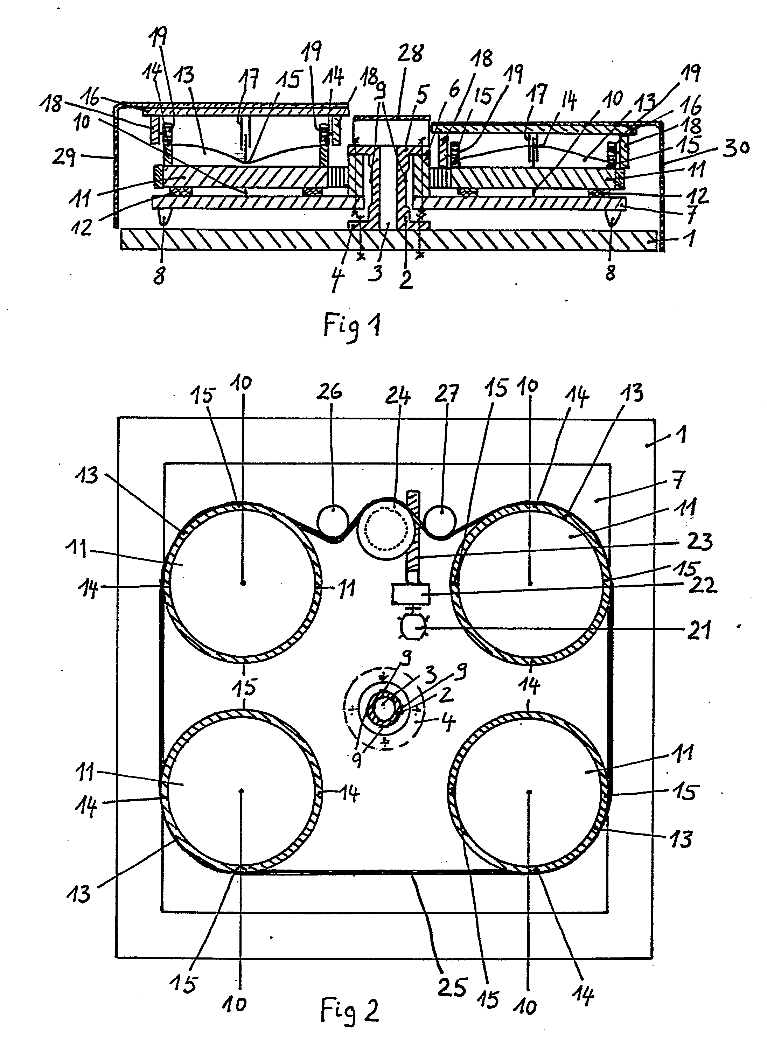

Die Erfindung ist anhand des in den Figuren 1 und 2 schematisch dargestellten Ausführungsbeispiels näher erläutert.

Es zeigen

Figur 1 einen Schnitt durch die erfindungsgemäße Vorrichtung

Figur 2 eine Draufsicht auf die erfindungsgemäße Vorrichtung. (teilweise geschnitten)

Die Vorrichtung besteht aus einer Basisplatte 1, auf der nahezu in der Mitte ein Biegestab 2 befestigt ist. Der Biegestab ist nach Art eines Doppelflansches ausgebildet und besitzt eine Bohrung 3. Der Biegestab 2 ist vorteilhafterweise durch spanende Bearbeitung z.B. durch Drehen aus dem vollen Material hergestellt. Die Befestigung des Biegestabes 2 auf der Basisplatte erfolgt durch nicht näher bezeichnete Schrauben im Bereich des unteren Flansches 4.

An der Unterseite des oberen Flansches 5 ist ein geteilter Distanzring 6 angeordnet, der über nicht dargestellte Schrauben mit der Standplatte 7 verbunden ist. Wird nun die Standplatte 7 einseitig belastet, verbiegt sich der Biegestab 2 leicht. Um eine "Überbiegung" des Biegestabes 2 zu vermeiden, sind zwischen Basisplatte 1 und Standplatte 7 mehrere Begrenzer 8 vorgesehen.

Um das Maß und die Richtung der Biegung des Biegestabes 2 zu erfassen, weist der Biegestab 2 vier angefaste Flächen auf, die um 90° versetzt zueinander zwischen den Flanschen 4 und 5 angefräst oder angefeilt sind und an denen Dehnungsmeßstreifen 9 befestigt sind. Die Dehnungsmeßstreifen 9 sind in nicht dargestellter Weise mit einer elektronischen Verstärkerschaltung verbunden.

Die Bohrung 3 im Biegestab 2 verleiht diesem ein höheres mechanisches Widerstandsmoment, so daß bei einseitiger Belastung die Dehnungsmeßstreifen und die elektronische Schaltung vor Überlastung geschützt ist. Der Biegestab 2 ist so bemessen, daß bei einer einseitigen Belastung der Standplatte 7 mit z.B. 100 kg die Standplatte 7 an ihrem Ende um ca. 1 mm aus der Horizontalen geneigt wird. Auf der Standplatte 7 sind vier Wellen 10 befestigt, auf denen je ein Zahnriemenrad 11 drehbar gelagert ist, welches sich über Ringe 12 aus selbstschmierendem druckfestem Material auf der Standplatte 7 abstützt. Die Zahnriemenräder 11 haben an ihrer Oberseite und zwar im Randbereich eine schiefe Ebene, die z.B. aus einem Ringstück 13 gebildet ist, welcher zwei um 180° versetzt zueinander angeordnete Hochpunkte 14 und um 90° versetzt zu den Hochpunkten 14 zwei Tiefpunkte 15. Zwischen den Tiefpunkten 15 und den Hochpunkten 14 nimmt die Höhe des Ringstückes 13 gleichmäßig zu. Die Hoch-bzw. Tiefpunkte 14 bzw. 15 auf einer Seite, d.h. in der Fig z.B. rechts, sind in der gleichen Stellung, wogegen die Hoch-bzw. Tiefpunkte 14 bzw. 15 auf der anderen Seite um 90° versetzt dazu angeordnet sind.

Über den Zahnriemenrädern 11 ist auf jeder Seite eine Fußplatte 16 angeordnet, die über geeignete Führungen 17 an den Wellen 10 geführt sind. An der Unterseite der Fußplatten 16 sind je vier Halterungen 18 befestigt, welche Rollen bzw. Kugellager 19 tragen, die wiederum sich auf den schiefen Ebenen bzw. dem Ringstück 13 abstützen. Über nicht näher bezeichnete Zugfedern sind die Fußplatten 16 mit der Standplatte 7 verspannt.

Der Antrieb der Zahnriemenräder 11 erfolgt durch einen Elektromotor 21, der über ein Getriebe 22 eine Spindel 23 antreibt, welche wiederum an ein Antriebsrad 24 angreift. Das Antriebsrad 24 weist an seiner Umfangsfläche eine der Verzahnung der Zahnriemenräder 11 entsprechende Verzahnung auf. In die Verzahnung des Antriebsrades 24 und der Zahnriemenräder 11 greift ein endloser Zahnriemen 25 ein, der mittels der Stellräder 26 und 27 vorgespannt wird.

Wird nun der Motor 21 betrieben, werden über das Getriebe 22, die Spindel 23,das Antriebsrad 24 und den Zahnriemen 25 die Zahnriemenräder 11 gleichmäßig angetrieben. Dadurch wälzen sich die Kugellager 19 auf den als schiefe Ebenen ausgebildeten Rohrstücken 13 ab und heben bzw. senken die Fußplatten 16. Dadurch, daß die Fußplatten 16 über die Führungen 17 an den Wellen 10 geführt sind, ist ein Verkanten vermieden. Die Führungen 17 sind an ihrer inneren Oberfläche mit einer selbstschmierenden Beschichtung versehen.

Die Vorrichtung ist noch mit einer Abdeckung versehen, von der das Mittelteil 28 an der Basisplatte 1 befestigt ist, wogegen die Seitenteile 29 und 30 auf den Fußplatten 16 aufliegen bzw. an diesen befestigt sind und deren Auf-und Abbewegungen mitmachen.

Die Basisplatte 1 kann noch in nicht dargestellter Weise auf einem ggfs. motorisch angetriebenen Drehgestell gelagert sein, welches eine Drehung um 90° ermöglicht, damit der Patient sowohl in der Frontalebene als auch in der Sagitalebene beobachtet bzw. vermessen werden kann.

Das Verhältnis der Drehzahl des Antriebsmotors 21 zur Drehzahl der Zahnriemenräder 11 ist in etwa 100:1, so daß durch die dadurch entstehende Hemmung eine Drehung der Zahnriemenräder 11 aufgrund einer Belastung der Fußplatten 17 verhindert wird.

Ein wesentlicher Vorteil der erfindungsgemäßen Vorrichtung besteht darin, daß bei einer eventuell auftretenden Störung der Antriebssteuerung diese nicht zerstört werden kann, da der Antrieb frei durchdrehen kann, infolge des Fehlens von Anschlägen. Dadurch kann man bei der Elektronik auf aufwendige Sicherheitsvorkehrungen gegen Fahren an einen Anschlag verzichten.

## Ansprüche

1. Vorrichtung zur Erzielung einer reproduzierbaren Körperhaltung des Menschen in freiem Stand, bei der die durch das Gewicht des Menschen erzeugten Kräfte auf Meßelemente übertragen werden, bestehend aus einer Basisplatte und einer über einen Biegestab mit der Basisplatte verbundenen Standplatte, wobei an dem Biegestab mindestens zwei Dehnungsmeßstreifen befestigt sind, dadurch gekennzeichnet, daß auf der Standplatte (7) zwei Fußplatten (16) höhenverstellbar nebeneinander angeordnet sind.

2. Vorrichtung nach Anspruch 1, dadurch gekennzeichnet, daß die Fußplatten (16) mittels - schiefer Ebenen (13) höhenverstellbar sind.

3. Vorrichtung nach Anspruch 1 oder 2, dadurch gekennzeichnet, daß auf der Standplatte (7) zumindest zwei Zahnräder oder Zahnriemenräder (11) drehbar gelagert sind, welche auf ihrer nach oben zeigenden Oberfläche zumindest eine am äußeren Umfang verlaufende schiefe Ebene (13) aufweisen und daß an der Unterseite der Fußplatten (16) mindestens zwei Räder oder Walzen (19) befestigt sind, welche sich bei Drehung der Zahn-riemenräder (11) auf den schiefen Ebenen (13) abwälzen.

4. Vorrichtung nach Anspruch 1 oder einem der folgenden, dadurch gekennzeichnet, daß für jede Fußplatte (16) zwei im Abstand zueinander angeordnete Zahnriemenräder (11) vorgesehen sind und im gleichen Abstand an der Unterseite der Fußplatte (16) je zwei Räder (19) angeordnet sind.

5. Vorrichtung nach Anspruch 3 oder 4, dadurch gekennzeichnet, daß die Räder (19) Kugellager sind, deren innerer Ring mit der jeweiligen Fußplatte (16) ver-bunden ist und deren äußerer Ring sich auf der jewei-ligen schiefen Ebene (13) abwälzt.

6. Vorrichtung nach Anspruch 1 oder einem der folgenden, dadurch gekennzeichnet,, daß die - schiefen Ebenen (13) als Bahnen im Bereich des Umfangs der Zahnriemenräder (11) verlaufen und zwei um 180° versetzt zueinander angeordnete Hochpunkte (14) und um 90° versetzt an den Hochpunkten (14) zwei Tiefpunkte (15) aufweisen und daß die Steigung der schiefen Ebene (13) zwischen den Tiefpunkten (15) und den Hochpunkten (14) konstant ist.

7. Vorrichtung nach Anspruch 1 oder einem der folgenden, dadurch gekennzeichnet, daß die Zahnriemenräder (11) von einem Antrieb (21,22,23) über einen gemeinsamen Zahnriemen (25) bzw. eine Kette angetrieben sind.

8. Vorrichtung nach Anspruch 1 oder einem der folgenden, dadurch gekennzeichnet, daß der Antrieb ein Elektro-motor (21) mit einem Getriebe (22) ist.

9. Vorrichtung nach Anspruch 1 oder einem der folgenden, dadurch gekennzeichnet, daß zumindest eines der Zahnriemenräder (11) eine zusätzliche Schneckenverzahnung aufweist, in welche eine vom Antrieb angetriebene Schnecke (23) eingreift.

10. Vorrichtung nach Anspruch 1 oder einem der folgenden, dadurch gekennzeichnet, daß ein gesondertes Zahnriemenrad (24) vorgesehen ist, welches über die Schnecke (23) angetrieben ist.

11. Vorrichtung nach Anspruch 1 oder einem der folgenden, dadurch gekennzeichnet, daß das Verhältnis der Drehzahl des Motors (21) und der Zahnriemenräder (11) zumindest 30:1, vorzugsweise größer ist.

12. Vorrichtung nach Anspruch 1 oder einem der folgenden, dadurch gekennzeichnet, daß der Biegestab (2) als Doppelflansch ausgebildet ist.

13. Vorrichtung nach Anspruch 1 oder einem der folgenden, dadurch gekennzeichnet, daß der Biegestab (2) hohl ausgebildet ist.

14. Vorrichtung nach Anspruch 1 oder einem der folgenden, dadurch gekennzeichnet, daß der Biegestab (2) ein rotationssymmetrischer Hohlkörper ist, der vorzugs-weise durch spanende Bearbeitung aus dem vollen Material hergestellt ist.

15. Vorrichtung nach Anspruch 1 oder einem der folgenden, dadurch gekennzeichnet, daß an dem Biegestab (2) zumindest zwei um 90° versetzt zueinander angeordnete, vorzugsweise vier gleichmäßig am Umfang verteilte plane Flächen angebracht sind, an welchen Dehnungs-meßstreifen (9) befestigt sind.

16. Vorrichtung nach Anspruch 1 oder einem der folgenden, dadurch gekennzeichnet, daß die Standplatte (7) so an dem Biegestab (2) befestigt ist, daß der Biegestab (2) nahezu lotrecht unter dem Schwerpunkt des auf den Fußplatten (16) stehenden Menschen gelegen ist.

17. Vorrichtung nach Anspruch 1 oder einem der folgenden, dadurch gekennzeichnet, daß die Fußplatten (16) beim Auf-und Niedergehen durch Führungen (17) geführt sind, in welche auf der Standplatte (7) befestigte Wellen (10) für die Zahnriemenräder (11) eindringen.

18. Vorrichtung nach Anspruch 1 oder einem der folgenden, dadurch gekennzeichnet, daß die Fußplatten (16) durch mindestens vier Zugfedern mit der Standplatte (7) verspannt sind.

Fig 1

Fig 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 712 294 (J.T. MULLER) * Zusammenfassung; Spalte 1, Zeilen 51-65; Spalte 3, Zeilen 7-36; Figuren 1-3 * | 1 | A 61 B 5/10 F 16 H 25/12 |
| A | | 12,15, 16 | |
| | --- | | |
| Y | FR-A-2 405 059 (D. IMBERNON) * Seite 1, Zeile 19 - Seite 2, Zeile 19; Seite 3, Zeilen 3-15; Seite 4, Zeilen 1-29; Seite 4, Zeile 40 - Seite 6, Zeile 3; Seite 6, Zeilen 19-24,32-36; Figuren 1-3 * | 1 | |
| A | | 8,17 | |
| | --- | | |
| A | US-A-2 095 268 (C.A. ROBERTS) * Seite 1, rechte Spalte, Zeilen 8-20,31-38; Seite 2, linke Spalte, Zeilen 32-40; Seite 2, linke Spalte, Zeile 51 - rechte Spalte, Zeile 17; Seite 2, rechte Spalte, Zeilen 55-67; Figuren 1,7-11 * | 1,2,17 | **RECHERCHIERTE SACHGEBIETE (Int. Cl.4)** |
| | --- | | A 61 B |
| A | FR-A-2 491 754 (L. DAVID) * Seite 1, Zeile 31 - Seite 2, Zeile 21; Seite 4, Zeilen 4-33; Seite 5, Zeile 30 - Seite 6, Zeile 27; Seite 7, Zeile 25 - Seite 8, Zeile 20; Seite 8, Zeile 37 - Seite 9, Zeile 9; Seite 9, Zeilen 26-29; Figuren 1-3 * | 1 | F 16 H |
| | --- | | |
| A | FR-A- 940 292 (R. VESSEREAU) * Seite 1, Zeilen 1-5,38-42; Seite 2, Zeilen 10-45; Seite 3, Zeilen 7-11; Figuren 1-3 * | 3,7 | |
| | --- -/- | | |

Der vorliegende Recherchenbericht wurde für alle Patentansprüche erstellt

| Recherchenort | Abschlußdatum der Recherche | Prüfer |
|---|---|---|
| DEN HAAG | 04-08-1988 | RIEB K.D. |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A- 831 875 (C.G.R.)<br>* Seite 3, Zeilen 19-43; Seite 4, Zeilen 74-78; Figur 4 *<br>--- | 3,4,7,8 | |
| A | FR-A-2 508 790 (SIEMENS AG)<br>* Seite 2, Zeilen 20-35; Seite 4, Zeilen 5-23; Figuren 3-5 *<br>--- | 3,4,7,8 | |
| A | FR-A- 539 227 (C.C.H. RIVIERE)<br>* Seite 1, Zeilen 12-16,45-52; Seite 2, Zeilen 12-28; Figuren 1,2 *<br>----- | 5 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 04-08-1988 | RIEB K.D. |